# EUROPEAN PATENT APPLICATION

(11) **EP 4 039 244 A1**
(43) Date of publication of application: **10.08.2022**
(21) Application number: 21155289.8
(22) Date of filing: 04.02.2021
(51) Int. Cl.: A61K 8/31, A61K 8/92, A61K 8/9789, A61Q 13/00

(54) **IMPROVEMENTS IN OR RELATING TO ORGANIC COMPOUNDS**

(71) Applicant: Givaudan SA, 1214 Vernier (CH)
(72) Inventor: The designation of the inventor has not yet been filed
(74) Representative: Global Patents

(57) **Abstract**

A method of suppressing or eliminating a sulphurous malodour associated with the generation of a thiol compound in a product comprising a composition containing a bio-sourced ingredient comprising ocimene and a container for holding and/or dispensing the composition, the thiol compound being generated as the result of interaction phenomena between the ocimene and the container.

## Description

### Field of the Invention

The present invention is concerned with methods and products for suppressing or eliminating unwanted or undesirable malodours that may be generated as a result of interaction phenomena between a bio-sourced ingredient and the container in which it is held, or from which it is dispensed, during its preparation, processing, distribution, storage or use.

### Background of the invention

In the normal life-cycle of consumer products, they will come into contact with many containers used in their preparation, processing, distribution, storage, and use. Interaction phenomena between consumer products and containers are known and can manifest themselves in many ways. For example, the structural integrity of containers can be compromised by ingredients contained in consumer products. Furthermore, the organoleptic properties of consumer products can be modified when materials in the container leach into the consumer product. Producers of consumer products are naturally concerned to anticipate and control any interaction phenomena that could damage containers, or otherwise lead to unwanted modifications of the taste, sight, smell or feel of their products.

Depending upon the industry sector, consumer products can include one or more fragrance ingredients, cosmetic active ingredients, flavour ingredients, ingredients that deliver a health, wellness or nutritional benefit, or functional ingredients, such as dyes and pigments, preservatives, antimicrobials, anti-oxidants, texturizers, emulsifiers or the like. Furthermore, with the trend towards naturality and health & well-being in the field of consumer products, it is increasingly common for many of these ingredients to be bio-sourced. Bio-sourced ingredients are ingredients that are extracted and/or derived from a natural source, such as plants, fungi, bacteria, algae or animal sources. They may be native, i.e. extracted unmodified from their natural state, or taken from their natural state and purified or even chemically modified. Bio-sourced ingredients are often complex substances containing many by-products in trace amounts, and this complexity can heighten the risk of unpredictable interaction phenomena.

When formulating with bio-sourced ingredients in the preparation of products, and particularly products intended for use in perfumery, cosmetics, and flavours, as well as the food & beverage, health, wellness & nutrition and pharma industries, there is an ongoing need to prevent, reduce or eliminate any undesirable organoleptic effects that might arise as a result of unpredictable interaction phenomena between bio-sourced ingredients and the containers in which they are held or from which they are dispensed.

### Summary of the Invention

When formulating with bio-sourced ingredients in the preparation of products useful in the fields of perfumery, cosmetics, flavours, food & beverage, health & well-being, nutrition and pharmaceuticals, applicant observed a surprising emanation of a sulphurous malodour reminiscent of a fermented, beer-like off-note. Furthermore, the generation of the off-note was intermittent, in the sense that it was typically observed when bio-sourced rather than synthetic ingredients were employed, and even then, not all bio-sourced ingredients were associated with the development of the sulphurous malodour.

After a complex and exhaustive analytical investigation the applicant could attribute the off-note to the generation of certain thiol compounds. More particularly, the applicant discovered in a surprising manner that certain bio-sourced ingredients contain malodour precursor compounds that can undergo chemical reactions with containers to produce thiol compounds that are implicated in the generation of the sulphurous malodour.

The invention provides in a first aspect a method of suppressing or eliminating a malodour from a product that comprises a composition containing a bio-sourced ingredient and a container for holding and/or dispensing the composition, wherein the bio-sourced ingredient contains an ocimene and wherein the method comprises at least one of the following steps:
Preventing the reaction of the ocimene with a co-reactant in the container that would lead to the generation of a thiol compound associated with a sulphurous malodour;
Removing from the composition any thiol compound generated as the result of the reaction of the ocimene with a co-reactant in the container;
Addition of a malodour-counteracting agent to the composition.

In a second aspect, the invention provides a product comprising a composition containing bio-sourced ingredient and a container for holding and/or dispensing the composition, wherein the bio-sourced ingredient contains an ocimene and the composition additionally comprises a malodour-counteracting agent.

In a third aspect, the invention provides a product comprising a composition containing a bio-sourced ingredient and a container for holding and/or dispensing the composition wherein the bio-sourced ingredient contains an ocimene and the container is free of any co-reactants that can react with an ocimene to generate a thiol compound associated with a sulphurous malodour.

The details, examples and preferences provided in relation to any one or more of the stated aspects of the present invention will be further described herein and apply equally to all aspects of the present invention. Any combination of embodiments, examples and preferences described herein below in all possible variations thereof are encompassed by the present invention unless otherwise indicated herein, or otherwise clearly contradicted by context.

### Detailed Description of the Invention

The present invention is based on the surprising discovery that certain bio-sourced ingredients, and in particular bio-sourced ingredients useful in perfumery, flavours, cosmetics, foods & beverages, health, wellness & nutrition, pharmaceuticals, as well as functional ingredients including preservatives, anti-oxidants, anti-microbial agents, colours, texturizers, emulsifiers and the like, may, as a result of their natural origins, contain ocimenes that can under certain conditions react with materials commonly used in containers to form thiol compounds, for example prenylthiol or 2-methyl-3-furanthiol, that are associated with the emanation of sulphurous malodours.

The generation of these thiol compounds in perfumery, flavours, cosmetics, food & beverage, health, wellness & nutrition and pharma applications as a result of interaction phenomena between bio-sourced ingredients and the containers used to hold and/or dispense them, was not heretofore known in the prior art. This surprising discovery underlying the present invention hereafter opens a way for the management, and in particular the suppression or elimination of sulphurous malodours that may be generated throughout the entire life-cycle of these bio-sourced ingredients or any product or composition containing them.

The term "container" as used herein refers to any vessel that is used to hold and/or dispense a composition containing a bio-sourced ingredient, during its preparation, processing, distribution, storage and use, and includes reaction vessels, bulk storage containers, packaging, dispensers, applicators, droppers, pumps including lotion pumps and spray pumps. Containers can comprise closure means and sealing means, such as valves, gaskets and the like, to prevent leakage from mating surfaces.

The compositions containing a bio-sourced ingredient are compositions useful in applications as diverse as perfumery, cosmetics, flavours, food & beverages, health & well-being, nutrition or pharmaceuticals. The compositions can be in the form of finished consumer products, that is, when held in a container they are in a finished form for sale to consumers for their own use or enjoyment. Particular categories of consumer products include those for use in fine perfumery, personal care, household care, laundry care, air care, and cosmetic treatment, as well as food & beverage products, products making a health & well-being claim, nutraceutical products and pharmaceutical products.

Particular examples of consumer products include alcoholic or non-alcoholic fine perfumes, household care, personal care or laundry care products, such as liquid detergents, powder detergents, fabric softeners, shampoos, shower gels, liquid soaps, dish wash products, conditioners, cleaners, body lotions, body creams, air fresheners, in-wash fabric perfumes or scent boosters, fabric freshener sprays, hygiene or care products, in particular in the range of body and hair care, cosmetic products, flavoured products, selected from the group consisting of foodstuffs, semi-luxury foods, snack foods, beverages, oral care products (e.g. oral hygiene products) nutraceuticals and products claiming health & well-being benefits, or pharmaceutical products.

The compositions can also be in a form intended for further production activity. That is, compositions can be perfume ingredients, perfume compositions, flavour ingredients, flavour compositions, or any functional ingredients or compositions containing same, such as preservatives, dyes or pigments, anti-microbial agents, anti-oxidants, texturizers, emulsifiers, or an ingredients that can deliver a health or wellness or nutritional effect, that may be used in, form a component part of, any of the foregoing consumer products.

The bio-sourced ingredients referred to herein contain an ocimene. In more particular embodiments of the invention the ocimene is alpha-ocimene, or the cis- or trans beta-ocimene, or mixtures thereof.

The ocimenes are examples of malodour precursor compounds that the applicant found in many bio-sourced ingredients and that have been surprisingly found to be capable of reacting with co-reactants found in containers to generate thiol compounds that are associated with a sulphurous malodour. However, owing to the complexity of bio-sourced ingredients, other trace compounds found in or derived from bio-sourced ingredients may also act as malodour precursor compounds. Accordingly, although hereafter reference is made specifically to ocimenes, the skilled person will understand that the reference to ocimenes can apply equally to other malodour precursor compounds referred to hereunder.

Other malodour precursor compounds may be C5-scaffolds including, but not limited to, the following structures, which may be found in bio-sourced ingredients, or which are produced when bio-sourced ingredients interact with co-reactants found in containers:

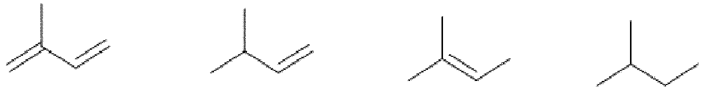

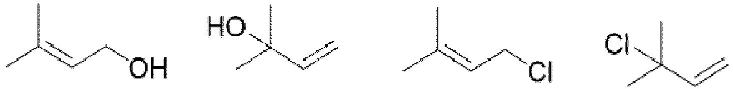

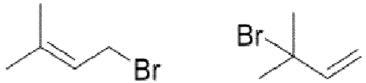

In accordance with the present invention, ocimenes have for the first time been identified as a materials with the propensity to produce thiol compounds implicated in the generation of sulphurous malodours under certain conditions referred to herein, and the invention resides the surprising discovery of this property of ocimenes and the means for either the prevention of interaction phenomena between ocimenes and co-reactants in containers, or the use of malodour-counteracting technology or other separation methods to suppress or eliminate the formation of the malodour. The co-reactants, which are described in more detail hereinbelow comprise vulcanized polymers or vulcanizing agents used in the preparation of such polymers. These vulcanizing agents and polymers are typically used in the formation of sealing means, such as gaskets used in containers, and particularly in valves and pumps used in dispensing means. More particularly, these vulcanizing polymers and agents comprise sulphur.

There are many sources of ocimenes in nature, and it is abundant in many plants and fruits. Particular bio-sourced ingredients according to the invention that contain ocimenes include, but are not limited to, the ingredient ocimene itself Tagete Oil; Oppoponax Oil; Neroli Oil; Lavender Oil; Angelica Root Oil; Lavandin Abrialis Oil; Angelica Seed Oil; Lime Oxide; Fennel Oil; Menthe Citrate; Petitgrain Oil; Basil Oil; Lentisque Oil; Mint Oils, including Spearmint Oil And Peppermint Oil; Lime Oil; Lemon Oil; Orange Oil; Mandarin Oil; Ho Leaf; Hops; Kumquat; Mango; Bigarde; Parsley Oil; Asafoetida Oil; Ay Oil; Basil Oil; Bay Oil; Bergamot Oil; Black Pepper Oil; Buchu Oil; Cananga Oil; Carrot Seed Oil; Celery Seed Oil; Chamomile Oil; Cinnamon Bark Oil; Clary Sage Oil; Coriander Seed Oil; Cubebe Oil; Davana Oil; Dill Seed Oil; Estragon Oil; Eucalyptus Oil; Fennel Oil; Geraniol Oil; Ginger Oil; Grapefruit Oil; Jasmin Absolute; Laurel Leaf Oil; Lemon Oil; Lime Oil; Lovage Root Oil; Marjoram Oil; Neroli Oil; Niaouli Oil; Nutmeg Oil; Oil Of Labdanum; Orange Flower Oil; Palmarosa Oil; Pennyroyal Oil; Peppermint Terpenes; Petitgrain Mandarine Oil; Pine Needle Oil; Cardamom Oil; Rosemary Oil; Shiso Oil; Spearmint Oil; Star Anise Oil; Valerian Oil; Vanilla Extract; and mixture thereof.

The exact mechanism of thiol compound generation is not entirely understood, however, it is believed that prenylthiol formation from ocimenes may proceed according to a process whereby oxidative or photolytic degradation products of ocimenes react with hydrogen sulphide or carbon disulphide to form prenylthiol directly, according to the mechanism provided below. Oxidation of ocimenes with oxygen (possibly in the presence of light) leads to formation of a peroxide, which undergoes Bayer-Villiger-like rearrangement to an oxonium compound. This is then hydrolysed to an aldehyde, which can undergo H-atom abstraction by a free radical (here a thiol radical) with concomitant decarbonylation to give the prenyl radical. This parallels the process observed in the natural generation of prenylthiol in beer from isohumulone. The prenyl radical can then trap thioethers to give prenylthiol and regenerate a thiol free radical catalyst.

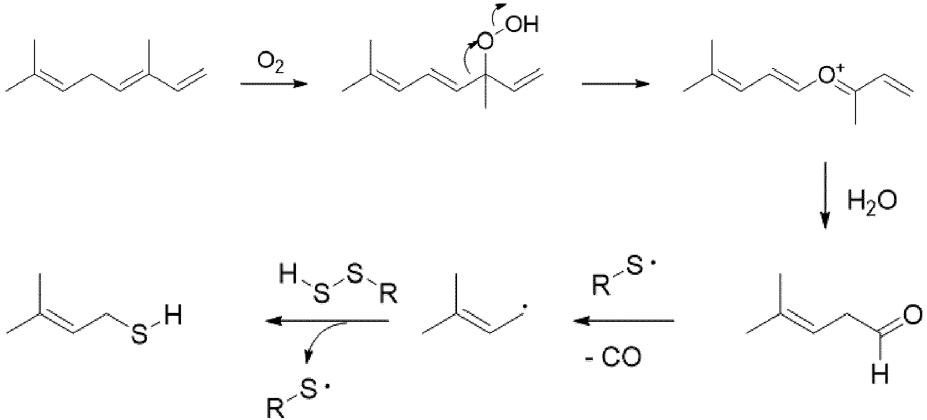

Alternatively, oxidative or photolytic degradation products of ocimenes can react with hydrogen sulphide or carbon disulphide to form or form a xanthate, which can rearrange to form prenylthiol:

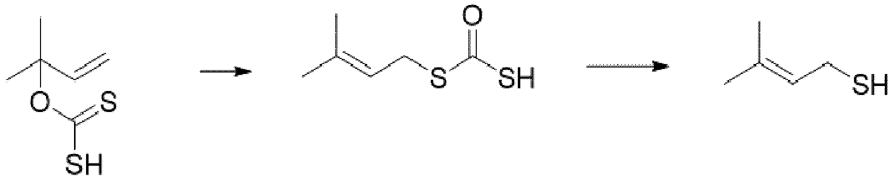

Another possible route involves the reaction of ocimenes with the vulcanizing agent S₂Cl₂ in a radical chain reaction via a radical species to give an intermediate that can undergo alkyl migration to provide a cation, followed by 1,5-H shift to provide an intermediate compound, which can be hydrolysed to give prenylthiol:

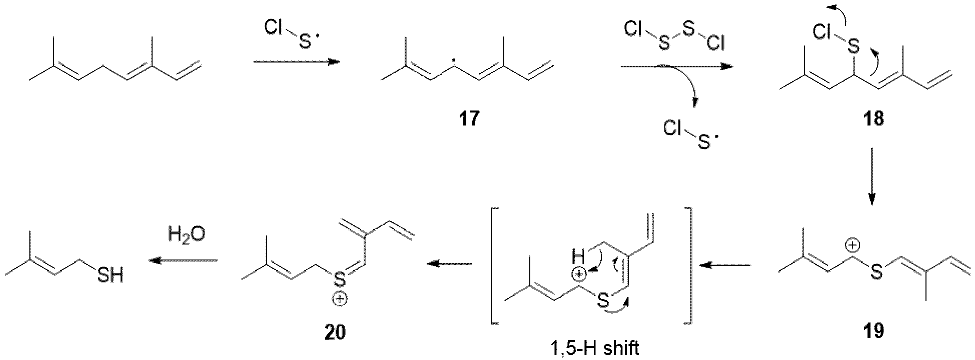

As for the malodorous thiol 2-methyl-3-furanthiol, it may be produced according to the following reaction scheme:

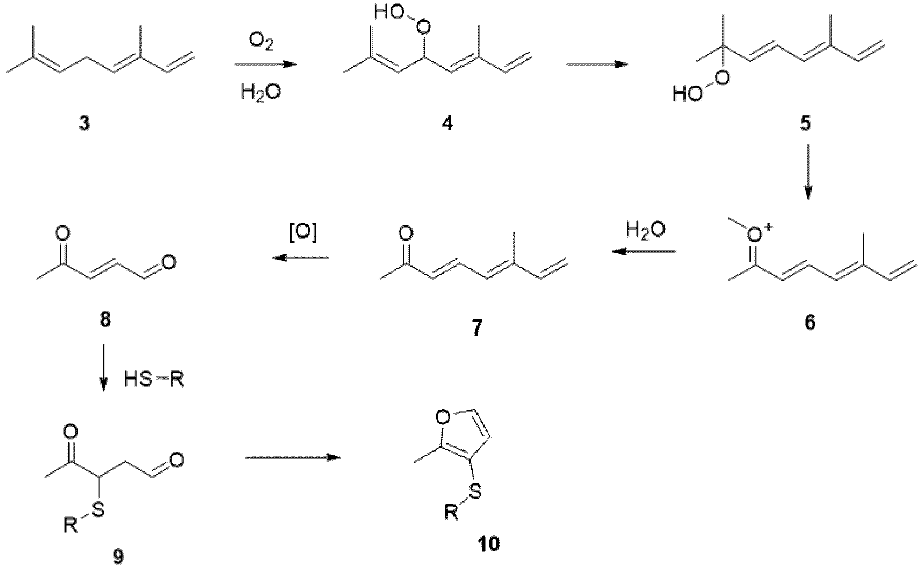

In addition to the role that an ocimene plays in the generation of thiol compounds, after its oxidation in air to a peroxide, it can become a strong electrophile trapping thiols (possibly under acid catalysis) and acting as a reservoir that can thermally or oxidatively release thiol compounds in a slow and sustained manner, exacerbating the malodour problem by contributing to its longevity.

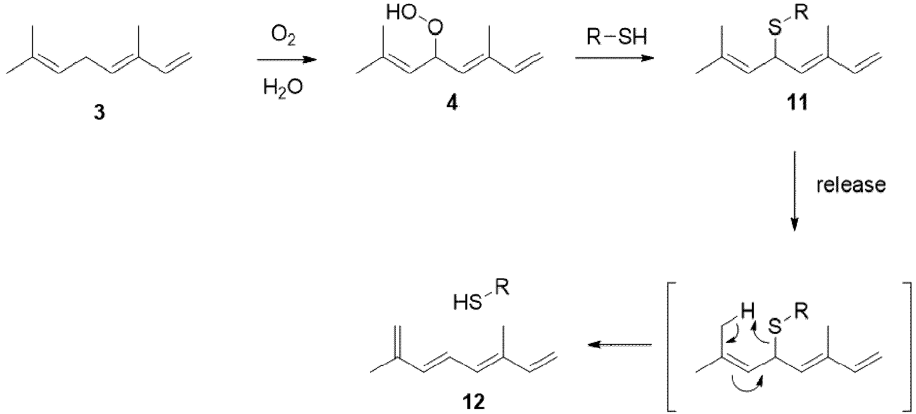

Certain bio-sourced ingredients referred to hereinabove, or more particularly the malodour precursor compounds they contain, most notably the ocimenes, have been shown to be a causative agent in the generation and release of thiol compounds, two of which have been identified for the first time. However, in accordance with the invention, the sulphurous malodour associated with these thiol compounds can be managed, that is, it can be suppressed or eliminated.

Applicant has identified several possible means for suppressing or eliminating the sulphurous malodours, which means may be used singularly or in combination.

In a particular aspect of the invention, malodour can be suppressed or eliminated by preventing the reaction of an ocimene with a co-reactant in the container that would lead to the generation of a thiol compound associated with a sulphurous malodour.

This can be achieved by selecting the container on the basis that it is free of any sulphur-containing vulcanized polymer or sulphur-containing vulcanizing agents used in the preparation of the polymer.

Sulphur-containing vulcanized polymers include, but are not limited to, polyisoprene (latex), polybutadiene, butadiene-ethylene-styrene copolymer, styrene-isoprene copolymer, BUNA (butadiene-acrylonitrile copolymer), BUNA-S (butadiene-styrene copolymer), BUTYL (isobutylene-isoprene copolymer); and EPDM (ethylene-propylene-diene), including dienes selected from ENB (ethylidene norbornene), DCPD (dicyclopentadiene) and VNB (vinyl norbornene). The vulcanizing agents are principally elemental sulphur and sulphur-containing compounds, such as sulphur-donating substances selected from compounds bearing disulphide groups, e.g. disulphur dichloride, or thiazoles, such as 2-mercaptobenzothiazole, thiurams, dithiocarbamates, xanthates and organic thioureas.

Accordingly, to the extent that the container comprises any plastics, rubber or polymer elements, they should not be vulcanized with sulphur-containing vulcanizing agents. Suitable materials include but are not limited to polymers vulcanized with peroxides, and non-vulcanized polymers such as polyethylene (PE); polypropylene (PP), polyethylene terephthalate (PET) and copolymers of PE and ethylene vinyl alcohol (EVOH).

Polyethylene (PE) in the context of the present invention, includes all of the different forms of PE classified by their differing density and branching, such as ultra-high-molecular-weight polyethylene (UHMWPE), ultra-low-molecular-weight polyethylene (ULMWPE or PE-WAX), high-molecular-weight polyethylene (HMWPE), high-density polyethylene (HDPE or PEAD), high-density cross-linked polyethylene (HDXLPE), cross-linked polyethylene (PEX or XLPE), medium-density polyethylene (MDPE), linear low-density polyethylene (LLDPE), low-density polyethylene (LDPE), very-low-density polyethylene (VLDPE), chlorinated polyethylene (CPE), preferably HDPE or LDPE. Similarly, polypropylene (PP), in the context of the present invention, also includes all of the different possible types of PP such as isotactic PP, syndiotactic PP, atactic PP, high crystalline PP (HcPP), random copolymers (RACO) of PP, block copolymers (HECO) of PP, uniaxially or biaxially oriented PP (BOPP). Polyethylene terephthalate (PET), in the context of the present invention, includes the different types of PET, such as polyethylenterephthalate and polyethylenterephthalate glycol.

Further particular embodiments of the present invention relate to the methods and products described herein, wherein the container is free of sulphur-containing vulcanized polymers or vulcanizing agents.

Containers typically comprise mating surfaces that generally require sealing means, such as a gasket to prevent leakage. If a container or part of the container such as a gasket does contain, or is formed from, a polymeric material then preferably it will be formed of polymers vulcanized with peroxides or non-vulcanized polymers such as polyethylene (PE), polypropylene (PP), polyethylene terephthalate (PET) and copolymers of PE and ethylene vinyl alcohol (EVOH), or mixtures thereof.

Accordingly, the invention provides in another aspect a product comprising a composition containing a bio-sourced ingredient comprising an ocimene and a container for holding and/or dispensing the composition, wherein the container is free of any sulphur-containing vulcanized polymer or sulphur-containing vulcanizing agents.

In another aspect of the invention there is provided a product comprising a composition containing a bio-sourced ingredient comprising an ocimene, and a container for holding and/or dispensing the composition, wherein the container comprises sealing means, such as a gasket, wherein the sealing means is free of sulphur-containing vulcanized polymers or sulphur-containing vulcanizing agents.

In particular embodiments of the invention, the sealing means, such as a gasket, is formed from polyethylene (PE), polypropylene (PP), polyethylene terephthalate (PET) and copolymers of PE and ethylene vinyl alcohol (EVOH), or mixtures thereof.

Ensuring that vulcanized polymers or vulcanizing agents are not used in containers represents an effective way of preventing the undesirable reaction of an ocimene with the container and prevents the formation of thiol compounds and associated sulphurous malodour. However, if it is not possible to prevent interactions between the ocimene and these co-reactants in the container, it is possible to suppress or even eliminate the formation of the thiol compounds and the associated sulphurous malodour by reducing the level of water present in the composition.

This is a particularly effective means of suppressing or eliminating malodour in compositions that are fine perfumery compositions. In fine perfumery compositions it is conventional to use a hydroalcoholic perfumery solvent (ethanol + water) that can contain levels of water as high as 15 wt %.

In accordance with the present invention there is provided a method of suppressing or eliminating a malodour from a product that comprises a fine perfumery composition containing a bio-sourced ingredient and a container for holding and/or dispensing the composition, wherein the bio-sourced ingredient contains an ocimene and wherein the method comprises the step of reducing the level of water in the composition to less than 3 wt %, and more particularly to 2 wt % or less.

The invention also provides in another of its aspects a product, comprising a fine perfumery composition comprising at least one perfume ingredient, a hydroalcoholic solvent, a bio-sourced ingredient comprising an ocimene, and a container for holding and/or dispensing the composition, wherein the composition comprises less than 3 wt %, and more particularly to 2 wt % or less of water.

Another means by which the sulphurous malodour is suppressed or eliminated in accordance with the present invention is by removing from the product any thiol compound generated as the result of the reaction of an ocimene with a co-reactant in the container.

Removal of thiol compounds from the composition can be achieved by separation means such as distillation or aeration, according to methods generally known in the art.

Alternatively or additionally, thiol compounds can be removed by a washing step. In particular embodiments, the thiol compounds can be removed by washing in the presence of a strong base, for example, a 2M NaOH solution. Alternatively or additionally, washing with an aqueous metal salt solution can be effective, e.g. washing with copper sulphate, or other suitable metal salt.

A further method of suppressing or eliminating a malodour from a product in accordance with the present invention comprises the step of adding a malodour-counteracting agent to the product.

Suitable malodour-counteracting agents include those that can mask malodour by superimposing it with a pleasant, stronger odour; those that cause cross-adaptation by blocking the malodour olfactory receptors; those that suppress malodour by creating a negative deviation in Raoult's law; those that can absorb the malodour in a porous or cage-like structure; and those that can eliminate or suppress malodour by chemical reaction.

The malodour-counteracting agents can be used singularly or in combination. The type and amount of agent one employs can be selected by the skilled person in a straightforward manner on the basis of the nature and intensity of the malodour that needs to be managed as well as the regulatory framework or the customer norms associated with the product into which the bio-sourced ingredient will be incorporated.

The malodour-counteracting agent will typically be mixed with the composition containing the bio-sourced ingredient. However, additionally, or alternatively, if the container comprises a vulcanized polymer, the malodour-counteracting agent can be incorporated into the vulcanized polymer.

In another aspect of the present invention, there is provided a product comprising an composition containing a bio-sourced ingredient comprising an ocimene, a malodour counteracting agent, and a container for holding and/or dispensing the composition, wherein the malodour-counteracting agent is selected from the group consisting of an agent that can mask the malodour by superimposing the malodour with a pleasant, stronger odour; an agent that causes cross-adaptation by blocking the malodour olfactory receptors; an agent that suppresses malodour by creating a negative deviation in Raoult's law; an agent that can absorb malodour in a porous or cage-like structure; and/or an agent that can eliminate or suppress malodour by chemical reaction with the thiol compound causing the malodour.

Malodour-counteracting agents capable of chemically reacting with thiol compounds to eliminate or suppress malodour may be selected from metal ions or non-odourant ingredients containing activated electrophilic groups that are capable of acting as Michael acceptors for thiol compounds.

Exemplary metal ion scavengers for thiol compounds include metal salts, such as zinc salts, and more particularly zinc carboxylates, e.g. zinc neodecanoate.

Non-odourant ingredients, that is, ingredients that are not primarily employed in products, such as perfumes or flavours, for their smell or aroma, that are effective Michael acceptors include the aromatic unsaturated carboxylic esters, such as those disclosed in WO 02/751788, including di-hexyl fumarate; alpha-beta unsaturated aldehyde, ketones or esters, as well as those ingredients in admixture with ammonium salts, such as those disclosed in WO 2012/126981; fumaric acid esters, such as those disclosed in U.S. Patent No. 3,077,457; and esters of alpha-, beta-unsaturated monocarboxylic acids, such as those disclosed in U.S. Patent No. 3,074,891. Any one or more of the foregoing ingredients are incorporated herein by reference as examples of suitable malodour counteracting agents.

Odourant ingredients, primarily of use as perfumery or flavour ingredients are likewise known to be useful malodour-counteracting agents, either by interacting chemically to trap malodour molecules or by masking them. Exemplary classes of ingredients include those that contain a carbon-carbon double bond conjugated with one or more carbonyl groups. Aldehydes are the most commonly used materials of this class for malodour counteractancy, such as trimethyl hexanal, other alkyl aldehydes, benzaldehyde, and vanillin; beta-dicarbonyl compounds, such as those disclosed in WO 2006/076821, including 2-(3,7-dimethyl-octa-2,6-dienylidene)-malonic acid diethyl ester; 3-octylidene-pentane-2,4-dione; 2-pyridin-2-ylmethylene-malonic acid diethyl ester; 2-octylidene-malonic acid dimethyl ester; 2-ethoxycarbonyl-but-2-enedioic acid diethyl ester; 2-acetyl-pent-2-enoic acid ethyl ester; 2-octylidene-malonic acid diethyl ester; 2-decylidene-malonic acid diethyl ester; 2-acetyl-dec-2-enoic acid ethyl ester; 2-(2-hydroxy-benzylidene)-1-phenyl-butane-1,3-dione; 2-(2-hydroxy-benzylidene)-malonic acid diethyl ester; 3-(2-hydroxy-benzylidene)-pentane-2,4-dione; 3-(2-hydroxy-4-methoxy-benzylidene)-pentane-2,4-dione; 2-(2-hydroxy-benzylidene)-3-oxo-butyric acid ethyl ester; 2-ethoxycarbonyl-oxymethylene-malonic acid diethyl ester; and 2-acetoxymethylene-malonic acid diethyl ester; ionones and other highly volatile odourant ingredients, such as those disclosed in US 5,919,440, all of which foregoing classes and specific examples of ingredients are incorporated by reference as malodour-counteracting agents in accordance with the present invention.

Particular examples of preferred odourant ingredients useful as malodour counteracting agents are selected from the group consisting of Adoxal (2,6,10-trimethylundec-9-enal); Decylenic Aldehyde (2-decenal, 9-decenal, (E)-2-decenal, (Z)-2-decenal, (E)-4-decenal, (Z)-4-decenal, (E)-6-decenal, (Z)-6-decenal, (E)-7-decenal, or (Z)-7-decenal); Undecylic Aldehyde MOA (2-methyldecanal); Undecylenic Aldehyde (undecanal); Lauric Aldehyde (dodecanal); Lauric Aldehyde MNA (2-methylundecanal); Hexyl Aldehyde (hexanal); Heptyl Aldehyde (heptanal); Octyl Aldehyde (octanal); Nonyl Aldehyde (nonanal); Decyl Aldehyde (decanal); Benzaldehyde (benzaldehyde); Bourgeonal (3-(4-(tert-butyl)phenyl)propanal); Cyclal C (2,4-dimethylcyclohex-3-ene-1-carbaldehyde); Cyclamen Aldehyde (3-(4-isopropylphenyl)-2-methylpropanal); Cyclohexal (4-(4-hydroxy-4-methylpentyl)cyclohex-3-enecarbaldehyde); Dupical ((E)-4-((3aS,7aS)-hexahydro-1H-4,7-methanoinden-5(6H)-ylidene)butanal); Fennaldehyde (3-(4-methoxyphenyl)-2-methylpropanal); Florhydral (3-(3-isopropylphenyl)butanal); Hexenal-2 Trans ((E)-hex-2-enal); Hydratropic Aldehyde (2-phenylpropanal); Lilial (3-(4-(tert-butyl)phenyl)-2-methylpropanal); Mefranal (3-methyl-5-phenylpentanal); Melonal (2,6-dimethylhept-5-enal); Methyl Cinnamic Aldehyde ((Z)-2-methyl-3-phenylacrylaldehyde); Scentenal ((3aR,4R,6S,7R,7aR)-6-methoxyoctahydro-1H-4,7-methanoindene-1-carbaldehyde); Para Tolyl Aldehyde (4-methyl-benzaldehyde); Tropional (3-(benzo[d][1,3]dioxol-5-yl)-2-methylpropanal); Isononyl Aldehyde (3,5,5-trimethylhexanal); Citral ((E)-3,7-dimethylocta-2,6-dienal); Citronellal (3,7-dimethyloct-6-enal); Trans-2-Decenal ((E)-dec-2-enal); Dodecenal ((E)-dodec-2-enal); Ethyl Citral ((2E,6E)-3,7-dimethylnona-2,6-dienal); Hydroxycitronellal (7-hydroxy-3,7-dimethyloctanal); Isocyclocitral (2,4,6-trimethylcyclohex-3-enecarbaldehyde); Isovaleraldehyde (3-methylbutanal); Jasmorange (2-methyl-3-(4-methylphenyl)propanal); Mahonial ((4E)-9-hydroxy-5,9-dimethyl-4-decenal); Melonia (7-methoxy-3,7-dimethyloctanal); Methoxymelonal (6-methoxy-2,6-dimethylheptanal); Methyl lonal Beta ((Z)-2-methyl-4-(2,6,6-trimethyl-1-cyclohex-2-enyl)but-2-enal); Methyl Phenyl Hexenal (5-methyl-2-phenylhex-2-enal, (E)-5-methyl-2-phenylhex-2-enal, or (Z)-5-methyl-2-phenylhex-2-enal); Myraldene (4-(4-methylpent-3-en-1-yl)cyclohex-3-enecarbaldehyde); Nonadienal ((2E,6Z)-nona-2,6-dienal); Nonenal-6-Cis ((Z)-non-6-enal); Nympheal (3-(4-(2-methylpropyl)-2-methylphenyl)propanal); Oncidal ((E)-2,6,10-trimethylundeca-5,9-dienal); Phenoxy Acetaldehyde (2-phenoxyacetaldehyde); Phenyl Propionic Aldehyde (3-phenylpropanal); Precyclemone B (1-methyl-4-(4-methylpent-3-en-1-yl)cyclohex-3-enecarbaldehyde); Safranal (2,6,6-trimethylcyclohexa-1,3-dienecarbaldehyde); Shisolia (4-vinylcyclohex-1-enecarbaldehyde); Silvial (2-methyl-3-[4-(2-methylpropyl)phenyl]propanal); Syringa Aldehyde (2-(p-tolyl)acetaldehyde); Tetrahydro Citral (3,7-dimethyloctanal); Tricyclal (2,4-dimethylcyclohex-3-enecarbaldehyde); Trans-2-Tridecenal ((E)-tridec-2-enal); Trifernal (3-phenylbutanal); Cetonal (2-methyl-4-(2,6,6-trimethylcyclohex-2-en-1-yl)butanal); Cinnamic Aldehyde ((2E)-3-phenylprop-2-enal); Vernaldehyde (1-methyl-4-(4-methylpentyl)cyclohex-3-enecarbaldehyde); and mixtures thereof.

Other odourant ingredients that can be employed in the present invention may be selected from the group consisting of Acetal R ((2-(1-propoxyethoxy)ethyl)benzene); Acetanisole (1-(4-methoxyphenyl)ethanone); Acetivenol ((4,8-dimethyl-2-propan-2-ylidene-3,3a,4,5,6,8a-hexahydro-1H-azulen-6-yl) acetate); Acetoin (3-hydroxybutan-2-one); Acetophenone (1-phenylethanone); Agarbois (N-ethyl-N-(m-tolyl)propionamide); Agrumex (2-(tert-butyl)cyclohexyl acetate); Akigalawood ((3-pentyloxan-4-yl) acetate); Aldrone (2-methyl-4-(5,6,6-trimethylbicyclo[2.2.1]hept-2-yl-cyclohexanone); Alicate (2,6-dimethylheptan-4-yl acetate); Allyl Amyl Glycolate (prop-2-enyl 2-(3-methylbutoxy)acetate); Allyl Caproate (prop-2-enyl hexanoate); Allyl Cyclohexyl Propionate (prop-2-enyl 3-cyclohexylpropanoate); Allyl Oenanthate (prop-2-enyl heptanoate); Alpinone (1-(2,4,4,5,5-pentamethylcyclopent-1-en-1-yl)ethan-1-one); Amberketal (3,8,8, 11a-tetramethyldodecahydro-1H-3,5a-epoxynaphtho[2,1-c]oxepine); Ambersage (2-methyl-2-(3-methylbutyl)-4,7-dihydro-1,3-dioxepine); Amyl Butyrate (pentyl butanoate); Amyl Caproate (pentyl hexanoare); Amyl Cinnamic Aldehyde ((Z)-2-benzylideneheptanal); Amyl Cinnamic Aldehyde Dimethyl Acetal ((Z)-2-benzylideneheptanal dimethylacetal); Amyl Phenyl Acetate (pentyl 2-phenylacetate); Anapear ((E)-methyl octa-4,7-dienoate); Anatolyl (phenylethyl 2-methylbutanoate); Anethole ((E)-1-methoxy-4-(prop-1-en-1-yl)benzene); Anther ((2-(isopentyloxy)ethyl)benzene); Aphermate (1-(3,3-dimethylcyclohexyl)ethyl formate); Azarbre (3,5-diethyl-2,5-dimethylcyclohex-2-enone); Azurone (7-isopentyl-2H-benzo[b][1,4]dioxepin-3(4H)-one); Benzophenone (diphenylmethanone); Benzyl Acetate; Benzyl Acetone (4-phenylbutan-2-one); Benzyl Butyrate (benzyl butanoate); Benzyl Cinnamate (benzyl 3-phenylprop-2-enoate); Benzyl Formate; Benzyl Isobutyrate (benzyl 2-methylpropanoate); Benzyl Isovalerate (benzyl 3-methylbutanoate); Benzyl Laurate (benzyl dodecanoate); Benzyl Methyl Ether ((methoxymethyl)benzene); Benzyl Phenyl Acetate (benzyl 2-phenylacetate); Benzyl Propionate (benzyl propanoate); Bergamyl Acetate (2-methyl-6-methyleneoct-7-en-2-yl acetate); Berryflor (ethyl 6-acetoxyhexanoate); Boisambrene Forte ((ethoxymethoxy)cyclododecane); Bornyl Acetate ((2S,4S)-1,7,7-trimethylbicyclo[2.2.1]heptan-2-yl acetate); Bornyl Acetate Liquid ((2S,4S)-1,7,7-trimethylbicyclo[2.2.1]heptan-2-yl acetate); Butyl Acetate; Butyl Butyrate (butyl butanoate); Butyl Butyro Lactate (1-butoxy-1-oxopropan-2-yl butanoate); Calypsone (6-methoxy-2,6-dimethyloctanal); Calyxol (ethyl 2-methyl-4-oxo-6-pentylcyclohex-2-ene-1-carboxylate); Camphor Synthetic ((1S,4S)-1,7,7-trimethylbicyclo[2.2.1]heptan-2-one); Carvone Laevo ((5R)-2-methyl-5-prop-1-en-2-ylcyclohex-2-en-1-one); Cashmeran (1,1,2,3,3-pentamethyl-2,3,6,7-tetrahydro-1H-inden-4(5H)-one); Cassione (4-(1-3-benzodioxol-5-yl)-2-butanone); Cedryl Acetate ((1S,6R,8aR)-1,4,4,6-tetramethyloctahydro-1H-5,8a-methanoazulen-6-yl acetate); Celery Ketone (3-methyl-5-propylcyclohex-2-enone); Cepionate (methyl 2-(3-oxo-2-pentylcyclopentyl)acetate); Cetone V ((E)-1-(2,6,6-trimethylcyclohex-2-en-1-yl)hepta-1,6-dien-3-one); Cinnamyl Acetate ((E)-3-phenylprop-2-en-1-yl acetate); Cinnamyl Cinnamate (3-phenylprop-2-enyl 3-phenylprop-2-enoate); Cis Jasmone ((Z)-3-methyl-2-(pent-2-en-1-yl)cyclopent-2-enone); Cis-3-Hexenyl Acetate ((Z)-hex-3-enyl acetate); Cistulate (N,2-dimethyl-N-phenylbutanamide); Citral Diethyl Acetal (1,1-diethoxy-3,7-dimethylocta-2,6-diene); Citral Dimethyl Acetal ((E)-1,1-dimethoxy-3,7-dimethylocta-2,6-diene); Citrathal R ((Z)-1,1-diethoxy-3,7-dimethylocta-2,6-diene); Citronellol (3,7-dimethyloct-6-en-1-ol) Citronellyl Acetate (3,7-dimethyloct-6-en-1-yl acetate); Citronellyl Butyrate (3,7-dimethyloct-6-en-1-yl butanoate); Citronellyl Ethoxalate (3,7-dimethyloct-6-en-1-yl ethyl oxalate); Citronellyl Isobutyrate (3,7-dimethyloct-6-en-1-yl 2-methylpropanoate); Citronellyl Nitrile (3,7-dimethyloct-6-enenitrile); Citronellyl Propionate (3,7-dimethyloct-6-en-1-yl propanoate); Citronitrile (3-methyl-5-phenylpent-2-enenitrile); Civettone ((Z)-cycloheptadec-9-enone); Claritone (2,4,4,7-tetramethyloct-6-en-3-one); Coniferan (2-(tert-pentyl)cyclohexyl acetate); Corylone (2-hydroxy-3-methylcyclopent-2-enone); Cristalon (ethyl 2,6,6-trimethylcyclohexa-1,3-diene-1-carboxylate); Cuminyl Acetate ((4-isopropylphenyl)methyl acetate); Cyclogalbanate (allyl 2-(cyclohexyloxy)acetate); Cyclohexyl Ethyl Acetate (2-cyclohexylethyl acetate); Cyclopidene (methyl 2-cyclopentylideneacetate); Cydrane (hexyl 2-methylbutanoate); Cyperate (3-tert-butyl-cyclohexyl acetate); Cyprisate (methyl 1,4-dimethylcyclohexanecarboxylate); Damascenone ((E)-1-(2,6,6-trimethylcyclohexa-1,3-dien-1-yl)but-2-en-1-one); Damascone Alpha ((E)-1-(2,6,6-trimethylcyclohex-2-en-1-yl)but-2-en-1-one); Damascone Beta ((E)-1-(2,6,6-trimethyl-1-cyclohexenyl)but-2-en-1-one); Decahydro Naphtyl Acetate Beta (1,2,3,4,4a,5,6,7,8,8a-decahydronaphthalen-2-yl acetate); Decahydro Naphtyl Formate Beta (1,2,3,4,4a,5,6,7,8,8a-decahydronaphthalen-2-yl formate); Decatone (6-isopropyloctahydronaphthalen-2(1H)-one); Decyl Acetate; Diethyl Malonate (diethyl propanedioate); Dihydro Ambrate (2-(sec-butyl)-1-vinylcyclohexyl acetate); Dihydro Anethole (1-methoxy-4-propylbenzene); Dihydro Farnesal ((Z)-3,7,11-trimethyldodeca-6,10-dienal); Dihydro lonone Beta (4-(2,6,6-trimethylcyclohex-1-en-1-yl)butan-2-one); Dihydro Jasmone (3-methyl-2-pentylcyclopent-2-enone); Dihydro Myrcenyl Acetate (2,6-dimethyloct-7-en-2-yl acetate); Dimethyl Anthranilate (methyl 2-(methylamino)benzoate); Dimethyl Benzyl Carbinyl Acetate (2-methyl-1-phenylpropan-2-yl acetate); Dimethyl Benzyl Carbinyl Butyrate (2-methyl-1-phenylpropan-2-yl butanoate); Dimethyl Octenone (4,7-dimethyloct-6-en-3-one); Dimethyl Phenyl Ethyl Carbinyl Acetate (2-methyl-4-phenylbutan-2-yl acetate); Dimyrcetol (2,6-dimethyloct-7-en-2-ol); Dione (2-(2-(3,3,5-trimethylcyclohexyl)acetyl)cyclopentanone); Diphenyl Oxide (oxydibenzene); Dispirone (7,9-dimethylspiro[5.5]undecan-3-one); Dodecalactone Delta (6-heptyltetrahydro-2H-pyran-2-one); Dodecalactone Gamma (5-octyloxolan-2-one); Elintaal (3-(1-ethoxyethoxy)-3,7-dimethylocta-1,6-diene); Ethyl Acetate; Ethyl Amyl Ketone (octan-3-one); Ethyl Benzoate; Ethyl Butyrate (ethyl butanoate); Ethyl Cinnamate (ethyl 3-phenylprop-2-enoate); Ethyl Decadienoate ((2E,4Z)-ethyl deca-2,4-dienoate); Ethyl Hexanoate; Ethyl Isoamyl Ketone (6-methylheptan-3-one); Ethyl Isobutyrate (ethyl 2-methylpropanoate); Ethyl Isovalerate (ethyl 3-methylbutanoate); Ethyl Linalool ((E)-3,7-dimethylnona-1,6-dien-3-ol); Ethyl Linalyl Acetate ((Z)-3,7-dimethylnona-1,6-dien-3-yl acetate); Ethyl Maltol (2-ethyl-3-hydroxy-4H-pyran-4-one); Ethyl Methyl-2-Butyrate (ethyl 2-methylbutanoate); Ethyl Octanoate;Ethyl Oenanthate (ethyl heptanoate); Ethyl Pelargonate (ethyl nonanoate); Ethyl Phenyl Acetate (ethyl 2-phenylacetate); Ethyl Phenyl Glycidate (ethyl 3-phenyloxirane-2-carboxylate); Ethyl Propionate (ethyl propanoate); Ethyl Safranate (ethyl 2,6,6-trimethylcyclohexa-1,3-diene-1-carboxylate); Ethyl Trans-2-Octenoate (ethyl (E)-oct-2-enoate); Eucalyptol ((1s,4s)-1,3,3-trimethyl-2-oxabicyclo[2.2.2]octane); Eugenol (4-allyl-2-methoxyphenol); Fenchone Alpha (1,3,3-trimethylbicyclo[2.2.1]heptan-2-one); Fenchyl Acetate ((2S)-1,3,3-trimethylbicyclo[2.2.1]heptan-2-yl acetate); Fixolide (1-(3,5,5,6,8,8-hexamethyl-5,6,7,8-tetrahydronaphthalen-2-yl)ethanone); Floralate ((2,4-dimethylcyclohex-3-en-1-yl)methyl acetate); Floralozone (3-(4-ethylphenyl)-2,2-dimethylpropanal); Florane (2-heptyloxolane); Floridile ((E)-undec-9-enenitrile); Floropal (2,4,6-trimethyl-4-phenyl-1,3-dioxane); Folione (methyl oct-2-ynoate); Fraistone (ethyl 2-(2,4-dimethyl-1,3-dioxolan-2-yl)acetate); Freskomenthe (2-(sec-butyl)cyclohexanone); Gardenol (1-phenylethyl acetate); Gardocyclene ((3aR,6S,7aS)-3a,4,5,6,7,7a-hexahydro-1H-4,7-methanoinden-6-yl 2-methyl propanoate); Georgywood (1-(1,2,8,8-tetramethyl-1,2,3,4,5,6,7,8-octahydronaphthalen-2-yl)ethanone); Geranyl Acetone ((E)-6,10-dimethylundeca-5,9-dien-2-one); Geranyl Crotonate ((E)-3,7-dimethylocta-2,6-dien-1-yl but-2-enoate); Geranyl Formate ((E)-3,7-dimethylocta-2,6-dien-1-yl formate); Geranyl Isobutyrate ((E)-3,7-dimethylocta-2,6-dien-1-yl 2-methylpropanoate); Geranyl Phenyl Acetate ((E)-3,7-dimethylocta-2,6-dien-1-yl 2-phenylacetate); Geranyl Propionate ((E)-3,7-dimethylocta-2,6-dien-1-yl propanoate); Geranyl Propionate ((E)-3,7-dimethylocta-2,6-dien-1-yl propanoate); Geranyl Tiglate ((E)-(E)-3,7-dimethylocta-2,6-dien-1-yl 2-methylbut-2-enoate); Givescone (ethyl 2-ethyl-6,6-dimethylcyclohex-2-enecarboxylate); Guaiyl Acetate (2-(3,8-dimethyl-1,2,3,4,5,6,7,8-octahydroazulen-5-yl)propan-2-yl acetate); Gyrane (2-butyl-4,6-dimethyl-3,6-dihydro-2H-pyran); Hedione (methyl 3-oxo-2-pentylcyclopentaneacetate); Heliotropyl Acetate (1,3-benzodioxol-5-ylmethyl acetate); Heptone (heptan-2-one); Herbanate ((2S)-ethyl 3-isopropylbicyclo[2.2.1]hept-5-ene-2-carboxylate); Herboxane (2-butyl-4,4,6-trimethyl-1,3-dioxane); Hexenyl Acetate Cis & Trans ((E)-hex-3-en-1-yl acetate); Hexenyl-3-Cis Butyrate ((Z)-hex-3-en-1-yl butanoate); Hexenyl-3-Cis Formate ((Z)-hex-3-en-1-yl formate); Hexenyl-3-Cis Hexenoate ((Z)-hex-3-en-1-yl (Z)-hex-3-enoate); Hexenyl-3-Cis Isobutyrate ((Z)-hex-3-en-1-yl 2-methylpropanoate); Hexenyl-3-Cis Methyl-2-Butyrate ((Z)-hex-3-en-1-yl 2-methyl butanoate); Hexenyl-3-Cis Propionate ((Z)-hex-3-en-1-yl propanoate); Hexenyl-3-Cis Tiglate ((Z)-hex-3-enyl] (E)-2-methylbut-2-enoate); Hexenyl-3-Trans Acetate ((E)-hex-3-enyl] acetate); Hexyl Acetate; Hexyl Butyrate (hexyl butanoate); Hexyl Isobutyrate (hexyl 2-methylpropanoate); Hexyl Methyl Butyrate/Cydrane (hexyl 2-methylbutanoate); Hexyl Propionate (hexyl propanoate); Homofuronol (2-ethyl-4-hydroxy-5-methylfuran-3(2H)-one); Hydratropic Aldehyde Dimethyl Acetal ((1,1-dimethoxypropan-2-yl)benzene); Hydroxycitronellal Dimethyl Acetal (8,8-dimethoxy-2,6-dimethyloctan-2-ol); lonone Beta ((E)-4-(2,6,6-trimethylcyclohex-1-en-1-yl)but-3-en-2-one); Irisantheme ((E)-3-methyl-4-(2,6,6-trimethylcyclohex-2-en-1-yl)but-3-en-2-one); Irone Alpha ((E)-4-(2,5,6,6-tetramethylcyclohex-2-en-1-yl)but-3-en-2-one); Irone F ((E)-4-(2,5,6,6-tetramethylcyclohex-2-en-1-yl)but-3-en-2-one); Iso E Super (1-(2,3,8,8-tetramethyl-1,2,3,4,5,6,7,8-octahydronaphthalen-2-yl)ethanone); Isoamyl Acetate (3-methylbutyl acetate); Isoamyl Butyrate (3-methylbutyl butanoate); Isoamyl Formate (3-methylbutyl formate); Isoamyl Propionate (3-methylbutyl propanoate); Isobornyl Propionate ((1S)-1,7,7-trimethylbicyclo[2.2.1]heptan-2-yl propanoate); Isobutyl Acetate (2-methylpropyl acetate); Isobutyl Isobutyrate (2-methylpropyl 2-methylpropanoate); Isobutyl Phenyl Acetate (2-methylpropyl 2-phenylacetate); Isodamascone (1-(2,4,4-trimethyl-1-cyclohex-2-enyl)but-2-en-1-one); Isojasmone (2-hexylcyclopent-2-enone); Isomenthone DI (2-isopropyl-5-methylcyclohexanone); Isononanyl Acetate (3,5,5-trimethylhexyl acetate); Isopentyl Isovalerate (3-methylbutyl 3-methylbutanoate); Isopentyrate (4-methylpent-4-en-2-yl 2-methylpropanoate); Isopropyl Methyl-2-Butyrate (isopropyl 2-methylbutanoate); Methyl lonones ((1E)-1-(2,6,6-trimethylcyclohex-1-en-1-yl)pent-1-en-3-one; (1E)-1-(2,6,6-trimethylcyclohex-2-en-1-yl)pent-1-en-3-one; (3E)-3-methyl-4-(2,6,6-trimethylcyclohex-1-en-1-yl)but-3-en-2-one; (3E)-3-methyl-4-(2,6,6-trimethylcyclohex-2-en-1-yl)but-3-en-2-one), or mixtures thereof; Jasmacyclene ((3aR,6S,7aS)-3a,4,5,6,7,7a-hexahydro-1H-4,7-methanoinden-6-yl acetate); Jasmatone (2-hexylcyclopentanone); Jasmopyrane (3-pentyltetrahydro-2H-pyran-4-yl acetate); Jasverate ([(2E)-2-ethylidene-5-bicyclo[2.2.1]heptanyl] propanoate); Jessate (ethyl 2-acetyloctanoate); Karanal (5-(sec-butyl)-2-(2,4-dimethylcyclohex-3-en-1-yl)-5-methyl-1,3-dioxane); Kefarene (1-(4-methoxy-2,2,6,6-tetramethylcyclohex-3-en-1-yl)ethanone); Kephalis (4-(1-ethoxyethenyl)-3,3,5,5-tetramethylcyclohexan-1-one); Ketoisophorone (2,6,6-trimethylcyclohex-2-ene-1,4-dione); Koavone ((Z)-3,4,5,6,6-pentamethylhept-3-en-2-one); Labienone ((E)-2,4,4,7-tetramethylnona-6,8-dien-3-one); Leaf Acetal ((Z)-1-(1-ethoxyethoxy)hex-3-ene); Lemonile ((2E,6Z)-3,7-dimethylnona-2,6-dienenitrile); Linalyl Acetate (3,7-dimethylocta-1,6-dien-3-yl acetate); Linalyl Cinnamate (3,7-dimethylocta-1,6-dien-3-yl 3-phenylprop-2-enoate); Linalyl Formate (3,7-dimethylocta-1,6-dien-3-yl formate); Linalyl Isobutyrate (3,7-dimethylocta-1,6-dien-3-yl 2-methylpropanoate); Linalyl Propionate (3,7-dimethylocta-1,6-dien-3-yl propanoate); Magnolione (3-(2-oxopropyl)-2-pentylcyclopentan-1-one); Maltol (3-hydroxy-2-methyl-4H-pyran-4-one); Maltyl Isobutyrate (2-methyl-4-oxo-4H-pyran-3-yl 2-methylpropanoate); Manzanate (ethyl 2-methylpentanoate); Melusat (ethyl 3,5,5-trimethylhexanoate); Menthanyl Acetate (2-(4-methylcyclohexyl)propan-2-yl acetate); Menthone (2-isopropyl-5-methylcyclohexanone); Mercapto-8 Menthene-1 Para (2-(4-methylcyclohex-3-en-1-yl)propane-2-thiol); Metambrate ((2-butan-2-yl-1-methylcyclohexyl) acetate); Methyl Acetophenone (1-(p-tolyl)ethanone); Methyl Amyl Ketone (heptan-2-one); Methyl Camomille (butyl 2-methylpentanoate); Methyl Cedryl Ketone (1-((1S,8aS)-1,4,4,6-tetramethyl-2,3,3a,4,5,8-hexahydro-1H-5,8a-methanoazulen-7-yl)ethanone); Methyl Cinnamate (methyl 3-phenylprop-2-enoate); Methyl Corylone (3,4-dimethylcyclopentane-1,2-dione); Methyl Cyclogeranate (methyl 2,6,6-trimethylcyclohex-2-ene-1-carboxylate); Methyl Dihydro Isojasmonate (methyl 2-hexyl-3-oxocyclopentane-1-carboxylate); Methyl Epi Jasmonate ((Z)-methyl 2-(3-oxo-2-(pent-2-en-1-yl)cyclopentyl)acetate); Methyl Heptenone (6-methylhept-5-en-2-one); Methyl Hexanoate (methyl hexanoate); Methyl Hexyl Ketone (octan-2-one); Methyl Jasmonate (methyl 2-(3-oxo-2-pent-2-enylcyclopentyl)acetate); Methyl Linoleate ((9E,12E)-methyl octadeca-9,12-dienoate); Methyl Methyl-2 Butyrate (methyl 2-methylbutanoate); Methyl Nonyl Ketone (undecan-2-one); Methyl Pamplemousse (6,6-dimethoxy-2,5,5-trimethylhex-2-ene); Methyl Phenyl Acetate (methyl 2-phenylacetate); Methyl Undecylenate (methyl (E)-undec-9-enoate); Methylionantheme Gamma ((E)-3-methyl-4-(2,6,6-trimethylcyclohex-1-en-1-yl)but-3-en-2-one); Muscenone ((Z)-3-methylcyclopentadec-5-enone); Muscone (3-methylcyclopentadecanone); Myraldyl Acetate ((4-(4-methylpent-3-en-1-yl)cyclohex-3-en-1-yl)methyl acetate); Myrascone (ethyl 2-ethyl-3,6,6-trimethylcyclohexene-1-carboxylate); Myrcene (7-methyl-3-methyleneocta-1,6-diene); Myrcenyl Acetate ((2-methyl-6-methylideneoct-7-en-2-yl) acetate); Nectaryl (2-(2-(4-methylcyclohex-3-en-1-yl)propyl)cyclopentan-1-one); Neobergamate Forte (2-methyl-6-methyleneoct-7-en-2-yl acetate); Neofolione ((E)-methyl non-2-enoate); Nerolidyl Acetate ((Z)-3,7,11-trimethyldodeca-1,6,10-trien-3-yl acetate); Nerolione (1-(3-methylbenzofuran-2-yl)ethanone); Nerone (1-(2-methyl-5-propan-2-yl-1-cyclohex-2-enyl)propan-1-one); Neryl Acetate ((Z)-3,7-dimethylocta-2,6-dien-1-ylacetate); Nonadienyl Acetate ((2Z,6E)-2,6-nonadien-1-ylacetate); Nonanone-2 (nonan-2-one); Nonyl Acetate (nonyl acetate); Nootkatone Crystals (4,4a-dimethyl-6-(prop-1-en-2-yl)-4,4a,5,6,7,8-hexahydronaphthalen-2(3H)-one); Nopyl Acetate (2-(6,6-dimethylbicyclo[3.1.1]hept-2-en-2-yl)ethyl acetate); Octenyl Acetate (oct-1-en-3-yl acetate); Octyl Acetate; Octyl Aldehyde Dimethyl Acetal (octanal dimethyl acetal); Opalal (7-isopropyl-8,8-dimethyl-6,10-dioxaspiro[4.5]decane); Orivone (4-(tert-pentyl)cyclohexanone); Oxyoctaline Formate (2,4a,5,8a-tetramethyl-1,2,3,4,4a,7,8,8a-octahydronaphthalen-1-yl formate); Pandanol ((2-methoxyethyl)benzene); Para Butyl Cyclohexyl Acetate (4-(tert-butyl)cyclohexyl acetate); Para Cresyl Acetate ((4-methylphenyl) acetate); Para Cresyl Caprylate ((4-methylphenyl) octanoate); Para Cresyl Isobutyrate ((4-methylphenyl) 2-methylpropanoate); Para Cresyl Phenyl Acetate ((4-methylphenyl) 2-phenylacetate); Para Cymene (p-cymene); Para Methyl Benzyl Acetate ((4-methylphenyl)methyl acetate); Para Tert Butyl Cyclohexanone (4-(tert-butyl)cyclohexanone); Paradisamide (2-ethyl-N-methyl-N-(m-tolyl)butanamide); Parmavert (1,1-dimethoxynon-2-yne); Peach Pure (5-heptyldihydrofuran-2(3H)-one); Pelargene (2-methyl-4-methylene-6-phenyltetrahydro-2H-pyran); Pepperwood (3,7-dimethylocta-1,6-dien-3-yl dimethylcarbamate); Peranat (2-methylpentyl 2-methylpentanoate); Petiole (2-propan-2-yloxyethylbenzene); Phantolide (1-(1,1,2,3,3,6-hexamethyl-2H-inden-5-yl)ethanone); Pharaone (2-cyclohexylhepta-1,6-dien-3-one); Phenoxy Ethyl Isobutyrate (2-(phenoxy)ethyl 2-methylpropanoate); Phenyl Ethyl Alcohol (2-phenylethanol); Phenyl Ethyl Butyrate (2-phenylethyl butanoate); Phenyl Ethyl Cinnamate (2-phenylethyl 3-phenylprop-2-enoate); Phenyl Ethyl Formate (2-phenylethyl formate); Phenyl Ethyl Isobutyrate (2-phenylethyl 2-methylpropanoate); Phenyl Ethyl Isovalerate (2-phenylethyl 3-methylbutanoate); Phenyl Ethyl Phenyl Acetate (2-phenylethyl 2-phenylacetate); Phenyl Ethyl Tiglate (phenethyl (E)-2-methylbut-2-enoate); Phenyl Propyl Acetate (3-phenylpropyl acetate); Piconia ((8aR)-4,4,8,8-tetramethylhexahydro-1H-3,8a-methanonaphthalen-5(6H)-one); Pivacyclene ((3aR,6S,7aS)-3a,4,5,6,7,7a-hexahydro-1H-4,7-methanoinden-6-yl 2,2-dimethylpropanoate); Pivarose (2,2-dimethyl-2-pheylethyl propanoate); Plicatone ((4aS,8aR)-7-methyloctahydro-1,4-methanonaphthalen-6(2H)-one); Poirenate (ethyl 2-cyclohexylpropanoate); Pomarose ((2E,5E)-5,6,7-trimethylocta-2,5-dien-4-one); Precarone ((1S,4R,6S)-4,7,7-trimethyl-4-(3-methylbut-2-en-1-yl)bicyclo[4.1.0]heptan-3-one); Prenyl Acetate (3-methylbut-2-en-1-yl acetate); Prunolide (5-pentyldihydrofuran-2(3H)-one); Quintone (2-pentylcyclopentanone); Raspberry Ketone (4-(4-hydroxyphenyl)butan-2-one); Resedal (2-(cyclohexylmethyl)-4,4,6-trimethyl-1,3-dioxane); Rhodinyl Acetate (mixture of 3,7-dimethyloct-6-en-1-ylacetate and 3,7-dimethyloct-7-en-1-yl acetate); Rhubofix ((2R,8aS)-3',6-dimethyl-3,4,4a,5,8,8a-hexahydro-1H-spiro[1,4-methanonaphthalene-2,2'-oxirane]); Rosacetol (2,2,2-trichloro-1-phenylethyl acetate); Rum Acetal (1,1-diethoxycyclohexane); Safraleine (2,3,3-trimethyl-1-indanone); Spirogalbanone Pure (1-(spiro[4.5]dec-6-en-7-yl)pent-4-en-1-one); Styrallyl Propionate (1-phenylethyl propanoate); Sylkolide ((E)-2-((3,5-dimethylhex-3-en-2-yl)oxy)-2-methylpropyl cyclopropanecarboxylate); Syvertal (2-(heptan-3-yl)-1,3-dioxolane); Tanaisone ((Z)-1-(cyclooct-3-en-1-yl)ethanone); Tangerinol ((E)-6,10-dimethylundeca-5,9-dien-2-yl acetate); Terpinolene (1-methyl-4-(propan-2-ylidene)cyclohex-1-ene); Terpinyl Acetate (2-(4-methyl-1-cyclohex-3-enyl)propan-2-ylacetate); Terpinyl Formate (2-(4-methyl-1-cyclohex-3-enyl)propan-2-yl formate); Tetrahydro Linalyl Acetate (3,7-dimethyloctan-3-yl acetate); Tetrascone (1-(4,4-dimethyl-2,3-dihydro-1H-naphthalen-1-yl)propan-1-one); Traseolide (1-(1,1,2,6-tetramethyl-3-propan-2-yl-2,3-dihydroinden-5-yl)ethanone); Tridecene-2-Nitrile ((E)-tridec-2-enenitrile); Veloutone (2,2,5-trimethyl-5-pentylcyclopentanone); Velvione ((Z)-cyclohexadec-5-enone); Veracetone (pent-4-enyl methyl ketone); Verdalia ((3aS,4R,6S,7R,7aR)-6-methoxy-3a,4,5,6,7,7a-hexahydro-1H-4,7-methanoindene); Vetikol Acetate/Corps Rhubarb (4-methyl-4-phenylpentan-2-yl acetate); Vetynal ((2R,5R,8S)-4,4,8-trimethyltricyclo[6.3.1.02,5]dodecan-1-ylacetate); Zinarine (2-(2,4-dimethylcyclohexyl)pyridine); and mixtures thereof.

Other odourant ingredients that can be employed in products of the present invention may also be selected from the group consisting of Allogal Base; Armoise Oil Morocco; Citronella Oil Java; Citronella Terpenes; Clary Sage Oil; Clove Leaf Oil; Eucalyptus Citriodora Oil; Fennel Oil; Grapefruit Oil; Lavandin Grosso Oil; Lemongrass Oil; Litsea Cubeba Oil; Armoise Oil Morocco High Thujone; Bergamot Oil; Camphor Oil; Cassis Base; Cedarwood Oil; Celery Seed Oil; Cinnamon Leaf Oil; Citronella Oil; Clary Sage Oil; Cucumber Base; Fir Oil; Galbanum Artessence Oil; Galbanum Oil; Grapefruit Terpenes; Nutmeg Oil; Olibanum Baumarome; Patchouli Oil; Prunal Base; Rhodinol Fraction Ex Geranium; Rosemary Base; Tamarine Base; Tea Tree Oil; Thyme Oil; Ylang Ylang Oil; Vanilla; or mixture thereof.

Although the aforementioned odourant ingredients can be used in products according to the present invention as malodour-counteracting agents, the applicant observed that products containing certain of these ingredients nevertheless developed the characteristic sulphurous malodour off-notes characteristic of the thiol compounds prenyl thiol or 2-methyl-3-furanthiol. Without intending to be bound by any particular theory, applicant believes that whereas many of the odourant ingredients useful as malodour-counteracting agents are Michael acceptors and are capable of chemically interacting with volatile sulphur compounds and trap them in the form of adducts, depending upon the strength of the Michael acceptor, the adducts can degrade thermally or oxidatively over time to release a thiol compound and create a lingering characteristic sulphurous malodour.

For example, products containing the odourant molecule Hedione, in particular, were found to be frequently associated with the sulphurous malodour. Hedione, a widely used odourant ingredient in many perfumery and flavour applications, contains 2-pentylcyclopent-2-en-1-one as an impurity. This impurity can act as a Michael acceptor to trap thiol compounds, and in particular prenylthiol, to form an adduct (see below), from which it can be slowly released over time. Prenyl thiol can also be trapped and subsequently released slowly over time by other odourant ingredients such as ionones and damascones. Adducts of 2-pentylcyclopent-2-en-1-one (left) and a damascone (right) are shown below:

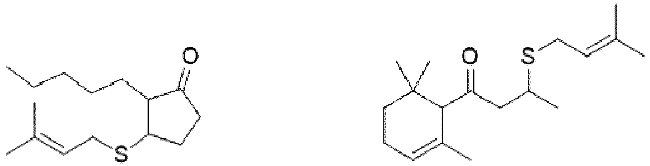

The observation that hedione-containing products appear to be particularly susceptible to the development of sulphurous malodour could also be a consequence of the fact that it is commonly used in large dosages in perfumery and flavour applications, and the use of this ingredient in large dosages in application, together with its physical properties, might push thiol compounds into the headspace and boost their negative sensorial effect.

This theory could also account for the observation that the off-notes are often observed too in products, and particularly products used in perfumery and flavours comprising ester odourant ingredients, which are ubiquitous in large dosages in perfumery and flavour compositions. Such esters include, but are not limited to Agrumex (2-(tert-butyl)cyclohexyl acetate); Allyl Heptanoate (prop-2-enyl heptanoate); Allyl Heptanoate (prop-2-enyl heptanoate); Amyl Salicylate (pentyl 2-hydroxybenzoate); Benzyl Acetate (benzyl acetate); Benzyl Phenyl Acetate (benzyl 2-phenylacetate); Benzyl Salicylate (benzyl 2-hydroxybenzoate); Bornyl Acetate ((2S,4S)-1,7,7-trimethylbicyclo[2.2.1]heptan-2-yl acetate); Cedryl Acetate ((1S,6R,8aR)-1,4,4,6-tetramethyloctahydro-1H-5,8a-methanoazulen-6-yl acetate); Citronellyl Acetate (3,7-dimethyloct-6-en-1-yl acetate); Cyclohexyl Salicylate (cyclohexyl 2-hydroxybenzoate); Dimethyl Benzyl Carbinyl Acetate (2-methyl-1-phenylpropan-2-yl acetate); Dimethyl Benzyl Carbinyl Butyrate (2-methyl-1-phenylpropan-2-yl butanoate); Ethyl Acetoacetate (ethyl 3-oxobutanoate); Ethyl Butyrate (ethyl butanoate); Ethyl Safranate (ethyl 2,6,6-trimethylcyclohexa-1,3-diene-1-carboxylate); Ethyl-2-Methyl Butyrate (ethyl 2-methylbutanoate); Eugenyl Acetate (4-allyl-2-methoxyphenyl acetate); Floralate ((2,4-dimethylcyclohex-3-en-1-yl)methyl acetate); Florocyclene ((3aR,6S,7aS)-3a,4,5,6,7,7a-hexahydro-1H-4,7-methanoinden-6-yl propanoate); Fraistone (ethyl 2-(2,4-dimethyl-1,3-dioxolan-2-yl)acetate); Fruitate ((3aS,4S,7R,7aS)-ethyl octahydro-1H-4,7-methanoindene-3a-carboxylate); Geranyl Acetate ((E)-3,7-dimethylocta-2,6-dien-1-yl acetate); Hedione (methyl 3-oxo-2-pentylcyclopentaneacetate); Helvetolide ([2-[1-(3,3-dimethylcyclohexyl)ethoxy]-2-methylpropyl]propanoate); Hexenyl-3-Cis Acetate ((Z)-hex-3-en-1-yl acetate); Hexenyl-3-Cis Salicylate ((Z)-hex-3-en-1-yl2-hydroxybenzoate); Hexyl Acetate (hexyl acetate); Hexyl Salicylate (hexyl 2-hydroxybenzoate); Isononanyl Acetate (3,5,5-trimethylhexyl acetate); Jasmacyclene ((3aR,6S,7aS)-3a,4,5,6,7,7a-hexahydro-1H-4,7-methanoinden-6-yl acetate); Linalyl Acetate (3,7-dimethylocta-1,6-dien-3-yl acetate); Manzanate (ethyl 2-methylpentanoate); Nerolidyle ((Z)-3,7,11-trimethyldodeca-1,6,10-trien-3-yl acetate); Para Tert Butyl Cyclohexyl Acetate (4-(tert-butyl)cyclohexyl acetate); Veramoss (methyl 2,4-dihydroxy-3,6-dimethylbenzoate); Vetiveryl Acetate ((4,8-dimethyl-2-propan-2-ylidene-3,3a,4,5,6,8a-hexahydro-1H-azulen-6-yl) acetate); and mixtures thereof.

Accordingly, in particular embodiments of the invention, products comprising a bio-sourced ingredient containing an ocimene; an odourant ingredient that can act, or contains impurities that can act, as a Michael acceptor, such as Hedione, damascones or ionones; or any of the ester odourant ingredients referred to hereinabove, should preferably also contain one or more of the non-odourant malodour counteracting agents referred to hereinabove.

The invention will be further explained and illustrated with reference to the following non-limiting examples.

### Example 1

### Preparation of Perfume Samples

A model perfume was prepared by dissolving 1% (w/w) Hedione (methyl dihydrojasmonate - obtained from Firmenich SA) in ethanol/water (85%/15% w/w) and labeled 'Perfume A'. 'Perfume A' was treated with either 0.04% (w/w) ocimene (mixture of isomers obtained from International Flavors and Fragrances), 0.04% (w/w) 'Lavender Oil France' (obtained from Givaudan SA), 0.04% (w/w) myrcene (obtained from Sigma-Aldrich), 0.04% of a stock solution of prenylthiol (CAS 5287-45-6, purchased e.g. from Sigma Aldrich) in Ethanol (concentration 25 ppm), or no additive. Samples were transferred into glass bottles and either closed with sprays containing different gasket types (see table below) or closed with a screw cap not containing vulcanized rubber gaskets. This resulted in the following perfume samples shown in Table 1, which were stored at 60 °C in an oven, for 7 days. Storage at elevated temperature was used to accelerate and accentuate the formation of the off-note. Samples stored at room temperature showed similar results but required longer storage times.

Gaskets made of different materials were obtained from Aptar Group Inc.: BUNA (Butadiene-acrylonitrile copolymer - vulcanized); Butyl (Isobutylene-Isoprene copolymer - vulcanized); ENB (ethylidene norbornene - vulcanized).

PE (polyethylene - not vulcanized) gasket was obtained from Gravis Orly.

**Table 1:**

| Sample Number | Additive (0.04% w/w) | gasket |
|---|---|---|
| 1 | Ocimene | none |
| 2 | Ocimene | Butyl |
| 3 | Ocimene | Buna + ENB |
| 4 | Ocimene | PE |
| 5 | Lavender Oil France | none |
| 6 | Lavender Oil France | Butyl |
| 7 | Lavender Oil France | Buna + ENB |
| 8 | Myrcene | Butyl |
| 9 | none | Butyl |
| 10 | Prenylthiol (25 ppm in Ethanol) | none |

### Analytical Techniques

### 1) GC-Sniffing

One microliter aliquots of the samples from Table 1 were manually injected and analyzed on mass spectrometer Agilent 5977B GC/MSD connected to a gas chromatograph Agilent 8890 GC System equipped with a split injector, an apolar HP-IMS capillary column (60 m, 0.32 mm internal diameter (i.d.), 0.25 µm film thickness) and a sniffing port (Givaudan in-house product). The carrier gas was helium, and the flow rate was set at 3.5 ml/min (constant flow mode). The split injector was maintained at 250 °C, and the split ratio was 10:1. The oven temperature was programmed to rise from 70 °C to 97 °C at 3 °C/min, then to 270 °C at 40 °C/min and held for 7 min. At the end of the capillary column, the effluent was split between the sniffing port and the MSD using deactivated fused silica capillaries (1.5 m, 0.15 mm i.d. to sniffing port and 0.65 m, 0.1 mm i.d. to MSD). Mass spectra were generated in electron ionization (EI) full scan mode (m/z range 30-450, 70 eV). Compound identification was made by comparing odour qualities, mass spectra and retention times with those of the respective reference compounds. Sensory evaluations at the sniffing port were performed by a trained panel of seven assessors.

### 2) GC-MS/MS

Aliquots (500 µl) of samples from Table 1 were dried by adding 1000 µl of MtBE and ∼450 mg MgSO₄ followed by filtration through a Na₂SO₄ cartridge. 1 µl of the dried sample was injected in a deactivated glass wool equipped liner at a split ratio of 1:10 using an inlet temperature of 230 °C. The GC-MS (Thermo Trace 1310 Series / Thermo TSQ 8000) was equipped with an Agilent J&W HP-5MS capillary column (15 m, 0.25 mm i.d. 0.25 µm film) operated at a constant He flow of 2 ml/min. The temperature program started at 35 °C (for 2 min), then with 20 °C/min to 75 °C followed by 40 °C/min to 270 °C (for 3 min). The MS transfer line and ion source temperatures were 250 °C and 200 °C, respectively. The triple quadrupole MS was equipped with an El ion source at 70 eV. An SRM detection method was used using the precursor ion mass of 102, collision energy of 10 and fragment ion mass of 69. Data was analyzed using Thermo Xcalibur 4.2.28.14.

### 3) Blotter Assessment

Blotter assessment of samples was conducted by dipping a paper blotter into the solution and allowing it to dry in the air for 5 seconds. The blotters were then smelled by a panel of three expert perfumers and characterized by the presence or absence of the characteristic prenylthiol off-note. In all cases tested, results from blotter evaluation were consistent with those observed in GC-Sniff analysis.

### Results

The samples described in Table 1 were analyzed using the Methods described above. The results are summarized in Table 2:

**Table 2:**

| Sample | off note perceived in sensory* | off note perceived in GC-Sniff* | prenylthiol detected** |
|---|---|---|---|
| 9 | no | no | no |
| 1 | no | no | no |
| 2 | yes | yes | yes |
| 10 | yes | yes | yes |
| 8 | no | not determined | not determined |
| 4 | no | not determined | not determined |
| 3 | yes | not determined | not determined |
| 5 | no | not determined | not determined |
| 6 | yes | not determined | not determined |
| 7 | yes | not determined | not determined |

| | | | |
|---|---|---|---|
| *sensory by panel form blotter and/or by GC-Sniff **detected by GC-Sniff and GC-EI MS/MS | | | |

### Example 2

### Preparation of Perfume Samples

A model perfume was prepared by dissolving 1% (w/w) Hedione (methyl dihydrojasmonate - obtained from Firmenich SA) and 0.04% (w/w) ocimene (obtained from International Flavors and Fragrances) in ethanol/water (85%/15% w/w) and labeled 'Perfume B'. 'Perfume C' was prepared by dissolving 70 ppb (parts per billion w/w) of prenylthiol (CAS 5287-45-6, purchased e.g. from Sigma Aldrich) in 'Perfume B'. Finally, 'Perfume C' was treated with 0.1% (w/w) dimethylfumarate (CAS 624-49-7 purchased e.g. from Sigma-Aldrich) to give 'Perfume D'.

### Blotter Assessment

Blotter assessment of Perfumes B, C and D was conducted by dipping paper blotters into each solution and allowing them to dry in the air for 5 seconds. The blotters were then smelled by a panel of five expert assessors and characterized by the presence or absence of the characteristic prenylthiol off-note, by consensus of the five panellists.

### Results

The Perfumes B, C and D described above were analyzed by blotter assessment. The results are summarized in Table 3:

**Table 3:**

| Sample | Odour description |
|---|---|
| Perfume B | light green floral; no off-note. |
| Perfume C | clear off-note; unpleasant, beer, fermented. |
| Perfume D | no off-note; not distinguishable from Perfume B. |

### Conclusion:

The addition of dimethylfumarate to a perfume containing the prenylthiol off-note acts as an efficient scavenger, removing the off-odor without otherwise disturbing the composition.

## Claims

1. A method of suppressing or eliminating a malodour from a product that comprises a composition containing a bio-sourced ingredient and a container for holding and/or dispensing the composition, wherein the bio-sourced ingredient contains an ocimene and wherein the method comprises at least one of the following steps:
- Preventing the reaction of the ocimene with a co-reactant in the container that would lead to the generation of a thiol compound associated with a sulphurous malodour;
- Removing from the composition any thiol compound generated as the result of the reaction of the ocimene with a co-reactant in the container;
- Addition of a malodour-counteracting agent to the composition.

2. A method according to claim 1, wherein the product is a perfumery product, a cosmetic product, a flavour product, a food & beverage product, a product delivering a health & wellness benefit, a nutraceutical product or a pharmaceutical product.

3. A method according to claim 1 or claim 2, wherein the composition is a perfume composition, a flavour composition, a cosmetic composition, a composition that can deliver a health & wellness benefit, a nutritional composition, a pharmaceutical composition, or a composition comprising a functional ingredient selected from a preservative, an anti-oxidant, an anti-microbial, a dye or pigment, a texturizer or an emulsifier.

4. A method according to any of the preceding claims, wherein the ocimene is alpha-ocimene, cis-beta ocimene, trans-beta ocimene, or a mixture thereof.

5. A method according to any of the preceding claims, wherein the bio-sourced ingredient is selected from the group consisting of ocimene, Tagete Oil; Oppoponax Oil; Neroli Oil; Lavender Oil; Angelica Root Oil; Lavandin Abrialis Oil; Angelica Seed Oil; Lime Oxide; Fennel Oil; Menthe Citrate; Petitgrain Oil; Basil Oil; Lentisque Oil; Mint Oils, including Spearmint Oil And Peppermint Oil; Lime Oil; Lemon Oil; Orange Oil; Mandarin Oil; Ho Leaf; Hops; Kumquat; Mango; Bigarde; Parsley Oil; Asafoetida Oil; Ay Oil; Basil Oil; Bay Oil; Bergamot Oil; Black Pepper Oil; Buchu Oil; Cananga Oil; Carrot Seed Oil; Celery Seed Oil; Chamomile Oil; Cinnamon Bark Oil; Clary Sage Oil; Coriander Seed Oil; Cubebe Oil; Davana Oil; Dill Seed Oil; Estragon Oil; Eucalyptus Oil; Fennel Oil; Geraniol Oil; Ginger Oil; Grapefruit Oil; Jasmin Absolute; Laurel Leaf Oil; Lemon Oil; Lime Oil; Lovage Root Oil; Marjoram Oil; Neroli Oil; Niaouli Oil; Nutmeg Oil; Oil Of Labdanum; Orange Flower Oil; Palmarosa Oil; Pennyroyal Oil; Peppermint Terpenes; Petitgrain Mandarine Oil; Pine Needle Oil; Cardamom Oil; Rosemary Oil; Shiso Oil; Spearmint Oil; Star Anise Oil; Valerian Oil; Vanilla Extract; and mixtures thereof.

6. A method according to any of the preceding claims wherein the thiol compound is prenylthiol or 2-methyl-3-furanthiol.

7. A method according to any of the preceding claims, wherein the malodour-counteracting agent is selected from the group consisting of an agent that can mask malodour by superimposing the malodour with a pleasant, stronger odour; an agent that causes cross-adaptation by blocking the malodour olfactory receptors; an agent that suppresses malodour by creating a negative deviation in Raoult's law; an agent that can absorb the malodour in a porous or cage- like structure; or an agent that can eliminate or suppress malodour by chemical reaction.

8. A method according to claim 7, wherein the malodour-counteracting agent that can eliminate or suppress malodour by chemical reaction is a Michael acceptor.

9. A method according to any of the preceding claims wherein the reaction of the ocimene with a co-reactant in the container is prevented by selecting a container that does not contain any sulphur-containing vulcanized polymer or any sulphur-containing vulcanizing agents.

10. A method according to any of the claims 1 to 8, wherein the reaction of the ocimene with a co-reactant is prevented by reducing the level of water in the composition.

11. A method according to claim 10, wherein the composition is a fine perfumery composition and the water level is less than 3 wt %, and more particularly 2 wt % or less.

12. A product comprising a composition containing a bio-sourced ingredient and a container for holding and/or dispensing the composition, wherein the bio-sourced ingredient contains an ocimene and wherein the container is free of any sulphur-containing vulcanized polymers or sulphur-containing vulcanizing agents.

13. A product according to claim 12, wherein the container comprises sealing means, such as a gasket, and wherein sealing means is free of any sulphur-containing vulcanized polymers or sulphur-containing vulcanizing agents.

14. A product accoring to claim 13, wherein the sealing means comprises a gasket formed of materials vulcanized with peroxides, polyethylene (PE); polypropylene (PP), polyethylene terephthalate (PET), and copolymers of PE and ethylene vinyl alcohol (EVOH).

15. A product according to any of the claims 12 to 14, wherein wherein the product is a consumer product selected from the group consisting of a fine perfumery product, personal care product, household care product, laundry care product, air care product, cosmetic product, food & beverage product, a product making a health & well-being claim, nutraceutical product and pharmaceutical product.

16. A product according to any of the claims 12 to 15, wherein the product is a fine perfumery product comprising a perfumery composition comprising at least one perfume ingredient and a hydroalcoholic solvent, optionally having a water content of less than 3 wt %, and more particularly 2 wt % or less.
